(19) Europäisches Patentamt
European Patent Office
Office européen des brevets

(11) EP 2 741 081 B1

# (12) EUROPEAN PATENT SPECIFICATION

(45) Date of publication and mention of the grant of the patent:
**10.04.2019 Bulletin 2019/15**

(51) Int Cl.:
***G01N 33/44*** *(2006.01)*

(21) Application number: **13193307.9**

(22) Date of filing: **18.11.2013**

## (54) Method for estimating vulcanization degree of rubber compound

Verfahren zur Schätzung des Vulkanisationsgrads einer Kautschukverbindung

Procédé d'estimation du degré de vulcanisation d'un composé de caoutchouc

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.12.2012 JP 2012267532**

(43) Date of publication of application:
**11.06.2014 Bulletin 2014/24**

(73) Proprietor: **SUMITOMO RUBBER INDUSTRIES, LTD.**
**Kobe-shi, Hyogo-ken, 651-0072 (JP)**

(72) Inventors:
- **Tsunoda, Masaya**
  **Kobe-shi,, Hyogo 651-0072 (JP)**
- **Yadav, Arjun**
  **Kobe-shi,, Hyogo 651-0072 (JP)**

(74) Representative: **Manitz Finsterwald**
**Patent- und Rechtsanwaltspartnerschaft mbB**
**Martin-Greif-Strasse 1**
**80336 München (DE)**

(56) References cited:
**US-A- 5 784 283**

- **ARRILLAGA ET AL: "Techniques used for determining cure kinetics of rubber compounds", EUROPEAN POLYMER JOURNAL, PERGAMON PRESS LTD. OXFORD, GB, vol. 43, no. 11, 29 October 2007 (2007-10-29), pages 4783-4799, XP022318833, ISSN: 0014-3057, DOI: 10.1016/J.EURPOLYMJ.2007.08.024**
- **OMRANI ET AL: "Cure kinetics, dynamic mechanical and morphological properties of epoxy resin-Im6NiBr2 system", EUROPEAN POLYMER JOURNAL, PERGAMON PRESS LTD. OXFORD, GB, vol. 44, no. 3, 31 December 2007 (2007-12-31), pages 769-779, XP022519367, ISSN: 0014-3057**
- **XIE M ET AL: "A new method to characterize the cure state of epoxy prepreg by dynamic mechanical analysis", THERMOCHIMICA ACTA, ELSEVIER SCIENCE PUBLISHERS, AMSTERDAM, NL, vol. 487, no. 1-2, 10 April 2009 (2009-04-10), pages 8-17, XP025970496, ISSN: 0040-6031, DOI: 10.1016/J.TCA.2009.01.001 [retrieved on 2009-01-15]**
- **RABEARISON N ET AL: "A FEM coupling model for properties prediction during the curing of an epoxy matrix", COMPUTATIONAL MATERIALS SCIENCE, ELSEVIER, AMSTERDAM, NL, vol. 45, no. 3, 1 May 2009 (2009-05-01), pages 715-724, XP026050159, ISSN: 0927-0256, DOI: 10.1016/J.COMMATSCI.2008.11.007 [retrieved on 2009-01-01]**
- **A. J. MARZOCCA ET AL: "Vulcanization kinetic of styrene-butadiene rubber by sulfur/TBBS", JOURNAL OF APPLIED POLYMER SCIENCE, vol. 101, no. 1, 1 January 2006 (2006-01-01), pages 35-41, XP055427564, US ISSN: 0021-8995, DOI: 10.1002/app.23173**
- **KAMAL M R: "THERMOSET CHARACTERIZATION FOR MOLDABILITY ANALYSIS", POLYMER ENGINEERING AND SCIENCE, BROOKFIELD CENTER, US, vol. 14, no. 3, 1 March 1974 (1974-03-01), pages 231-239, XP000576769, DOI: 10.1002/PEN.760140312**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

Background of the Invention

The present invention relates to a method for accurately estimating the vulcanization degree of a rubber compound, in particular, a change curve of the vulcanization degree of the rubber compound with vulcanizing time.

when manufacturing a rubber product such as vehicle tire, it is important to estimate a change in the vulcanization degree of a rubber compound from moment to moment in order to determine and control the vulcanizing temperature and vulcanizing time.

In the following non-patent document 1, it is disclosed that, the following expression (3) is integrated so that the expression (3) is approximated to an actual vulcanization curve which shows the vulcanization degree or torque of a rubber compound against the vulcanizing time, and
by the use of such approximated expression (3), the vulcanization degree X is estimated.

$$\frac{dX}{dt} = (K_1 + K_2 X^m)(1+X)^n \qquad \text{Expression (3)}$$

wherein

X: vulcanization degree
t: vulcanizing time
K1, K2, m, n: invariables.

Non-patent document 1:
W.J.TOTH, J.P.CHANG and C.ZANICHELLI, "Finite element evaluation of the state of cure in a tire", Tire Science and Technology: October 1991, vol. 19, No. 4, pp. 178-212.

In the non-patent document 1, in order to enable the integration of the expression (3), fixed real numbers are assigned to the invariables m, n and K1, and
only the value of the invariable K2 is determined so that the expression (3) becomes close to the actual vulcanization curve as far as possible. Therefore, the method disclosed in the non-patent document 1 has its limit, and the vulcanization degree of the rubber compound can not be accurately estimated.

In order to make an accurate estimation, it is conceivable to determine the values of the invariables m, n, K1 and K2 in the expression (3) so that the obtained expression becomes more close to the actual vulcanization curve. In this case however, it is complex and difficult to determine the values suitable for the invariables m, n, K1 and K2, and it is not assured that suitable values can be found.

Arrilaga et al. disclose in European Polymer Journal 43 (2007) 4783-4799 the expression $\frac{d\alpha}{dt} = (k_1 + K_2 \cdot \alpha^m) \cdot (1-\alpha)^n$, wherein $\alpha$ is the cure degree and t is the time. Plots obtained from oscillating disc rheometer (ODR), moving die rheometer (MDR) and differential scanning calorimeter (DSC) measurements were used by Arrilaga et al. to determine constants for the Isayev model.

Omrani et al. describe in European Polymer Journal 44 (2008) 769-779 effects of a Nickel-imidazole catalyst on the cure kinetics of an epoxy resin.

## Summary of the Invention

It is therefore, an object of the present invention to provide a method for accurately estimating the vulcanization degree of a rubber compound.

According to the present invention, a method for estimating the vulcanization degree of a rubber compound according to claim 1, comprises
a measuring process in which a measured vulcanization curve which is a change curve of the vulcanization degree of the rubber compound with vulcanizing time is obtained by actually measuring the shearing resistance (or torque) of the rubber compound during vulcanization,
an invariable determining process in which values of the invariables K1, K2 and m of the following expression (1) are determined which values minimize the error between the measured vulcanization curve and an estimated vulcanization curve which is a curve of the expression (1),

$$\ln\left[\frac{1}{(1-X)}\frac{dX}{dt}-K_1\right]=\ln(K_2)+m\ln(X) \qquad \text{Expression (1)}$$

wherein

X: vulcanization degree
t: vulcanizing time

wherein
the invariable determining process comprises
an invariable calculating process in which each of different real numbers is assigned to the invariable K1 of the expression (1), wherein the real numbers are more than 0 and less than 1, and
a linear expression, whose gradient and intercept are equal to m and K2 respectively, is transformed from the expression (1) and is approximated to the measured vulcanization curve, and then from the approximated expression, a set of values of the invariables K1, K2 and m are obtained,
whereby plural sets of the values corresponding to the respective real numbers are obtained, and
a fitted curve determining process in which a plurality of the estimated vulcanization curves are obtained by assigning the plural sets of the values to the expression (1), and
from a plurality of the estimated vulcanization curves, an estimated vulcanization curve whose error from the measured vulcanization curve is smallest, is found out.

The above and further objects and novel features of the present invention will more fully appear from the following detailed description when the same is read in connection with the accompanying drawing.

**Brief Description of the Drawings**

Fig. 1 shows a flow chart of a method for estimating the vulcanization degree of a rubber compound as an embodiment of the present invention.
Fig. 2 is a graph showing the measured vulcanization curve and estimated vulcanization curves at a vulcanizing temperature of 150 degrees C.
Fig. 3 is a graph showing the measured vulcanization curve and estimated vulcanization curves at a vulcanizing temperature of 160 degrees C.
Fig. 4 is a graph showing the measured vulcanization curve and estimated vulcanization curves at a vulcanizing temperature of 170 degrees C.
Fig. 5 is a graph showing the measured vulcanization curve and estimated vulcanization curves at a vulcanizing temperature of 180 degrees C.
Fig. 6 is a flow chart of the invariable determining process in this embodiment.
Fig. 7 is a flow chart of the invariable calculating process in this embodiment.
Fig. 8 is a flow chart of the fitted curve determining process in this embodiment.
Fig. 9(a) is a graph of the invariable K1 and vulcanizing temperature.
Fig. 9(b) is a graph of the invariable K2 and vulcanizing temperature.
Fig. 9(c) is a graph of the invariable m and vulcanizing temperature.
Fig. 10 shows an example of the change curve of the vulcanizing temperature with vulcanizing time used in a process of vulcanizing a rubber compound.
Fig. 11 is a flow chart of the estimating process in this embodiment.
Fig. 12 shows an estimated vulcanization curve obtained through the estimating process.

Description of the Preferred Embodiments

Embodiments of the present invention will now be described in detail in conjunction with accompanying drawings.
The method for estimating the vulcanization degree of a rubber compound according to the present invention can be suitably used for rubber compounds constituting a rubber product such as vehicle tire.
Fig. 1 shows a flow chart of the estimating method as an embodiment of the present invention.

* Measuring process S1

In the measuring process S1, the shearing resistance or torque of the rubber compound during vulcanization is actually measured to obtain the vulcanization curve (hereinafter the measured vulcanization curve or actual vulcanization curve). Incidentally, the vulcanization curve shows the vulcanization degree X of the rubber compound as a function of the vulcanizing time t.
In this embodiment, in order to measure the torque T of the rubber compound, a so called Curelastometer, a rubber testing machinery for assessing vulcanization characteristics, is employed.
As shown in Fig. 2 to Fig. 5, such torque is measured at different vulcanizing temperatures, for example, 150, 160, 170 and 180 degrees C.
The measuring conditions are as follows.
measuring method: JIS K 6300, Physical Testing Methods
For unvulcanized Rubber
amplitude angle: +/-1 degrees
number of torsional vibration: 100/minutes
For example, a curelastometer 7(seven), a product of JTR Trading Co., Ltd. is used.
Form the measured torque T, the vulcanization degree X of the rubber compound is calculated by the use of the following expression (4).

$$X(t) = \frac{T - T_{\min}}{T_{\max} - T_{\min}} \qquad \text{Expression (4)}$$

wherein

$T_{max}$: torque of rubber compound after vulcanization
$T_{min}$: torque of rubber compound before vulcanization

The vulcanization degree X indicates the degree (from 0 to 1) of completion of the vulcanization. when the torque T is minimum ($T_{min}$), the vulcanization degree X=0. when the torque T is maximum ($T_{max}$), the vulcanization degree X=1.
For each of the vulcanizing temperatures, the vulcanization degree X of the rubber compound is plotted against the vulcanizing time t to obtain the measured vulcanization curve 2 as shown in Fig. 2 to Fig. 5.

* Invariable determining process S2

In the process S2, the following expression (1) is used.

$$\ln\left[\frac{1}{(1-X)}\frac{dX}{dt} - K_1\right] = \ln(K_2) + m\ \ln(X) \qquad \text{Expression (1)}$$

wherein

X: vulcanization degree
t: vulcanizing time
K1, K2, m: invariables.

The expression (1) is obtained from the expression (3) explained in the non-patent document 1. 1 (one) is assigned to the invariable n of the expression (3), and the expression (3) is converted to natural logarithm, and then the converted expression is transformed to the expression (1).
In the invariable determining process S2, values of the invariables K1, K2 and m of the expression (1) are determined which values can minimize the error between the measured vulcanization curve 2 and the estimated vulcanization curve 3 which is the curve of the expression (1).
Fig. 6 shows a flowchart of the invariable determining process S2 in this embodiment.

** Invariable calculating process S21

For each of the vulcanizing temperatures, in this example, 150, 160, 170 and 180 degrees C, at which the measured vulcanization curves 2 were obtained, the following are performed:

a plurality of different real numbers are assigned to the invariable K1 of the expression (1), and
for each of the different real numbers for K1,
values of the invariables K2 and m are determined.

A flowchart of this invariable calculating process S21 is shown in Fig. 7.

*** Process S211

First, the lower limit of the real number or the smallest real number is assigned to the invariable K1 of the expression (1). For example, the lower limit of the real number is $1X10^{-10}$.
Therefore, the expression (1) can be transformed into a linear expression whose gradient and intercept are equal to m and K2, respectively.

*** Process S212

Next, the linear expression transformed from the expression (1) is approximated to the measured vulcanization curve 2 at the vulcanizing temperatures concerned (for example 150 degrees C).
For example, the method of least squares is used to approximate the linear expression 4 to the measured vulcanization curve 2 as shown in Fig. 2. Thus, the approximation is possible without complex calculation.

*** Process S213

Next, from the linear expression 4 approximated to the measured vulcanization curve 2 in the process S212, the values of the invariables K1, K2 and m thereof are obtained. The invariable K2 and invariable m can be obtained as the gradient and intercept of the approximated linear expression 4. Thus, in this process 213, a set of values of the invariables K1, K2 and m, corresponding to the above-mentioned real number assigned to the invariable K1 in the process S211, can be obtained.

*** Process S214

Next, it is judged if the current value of the invariable K1 is equal to or more than its upper limit. Here, the upper limit is 1. Thus, the real numbers assigned to the invariable K1 are more than 0 and less than 1. If "yes", then the procedure goes to process S216.
If "no" namely the current value of the invariable K1 is less than the upper limit, then the procedure goes to process S215.

*** Process S215

In the process S215, the invariable K1 is increased to a value equal to the current value multiplied by a predetermined incremental modulus L. In this embodiment, the incremental modulus L is 1.1.
Then, the processes S212 to S214 are repeated.
Thus, by the processes S211-S215, with respect to the concerned vulcanizing temperature, a set of values of the invariables K1, K2 and m are obtained for each of the real numbers from the lower limit to the upper limit assigned to K1. In other words, plural sets of values of K1, K2 and m corresponding to the respectively real numbers are obtained with respect to the concerned vulcanizing temperature.
In Fig. 2, only one example of the linear expression 4 approximated to the measured vulcanization curve 2 is shown. But, a plurality of the approximated linear expressions 4 exist corresponding to the real numbers.
By limiting the real numbers assigned to the invariable K1 in a range of more than 0 and less than 1, the linear expression 4 transformed from the expression (1) can be well approximated to the measured vulcanization curve 2. If the real number is more than 1, the degree of the approximation is not increased, and the computational cost is increased.

*** process S216

In the process S216, it is judged if the above-mentioned plural sets of values of the invariables K1, K2 and m have been obtained with respect to every vulcanizing temperature, in this example, 150, 160, 170 and 180 degrees C. If "yes", then the procedure goes to a fitted curve determining process S22.
If "no", namely, there exists a vulcanizing temperature about which the plural sets of values of the invariables K1, K2 and m are not yet obtained, then with respect to that vulcanizing temperature, the processes S211 to S216 are repeated.
Therefore, in the invariable calculating process S21, as shown in Fig. 2 to Fig. 5, with respect to every vulcanizing temperature, the linear expression 4 is approximated to the measured vulcanization curve 2, and plural sets of values of the invariables K1, K2 and m are obtained.

** Process S22

After the invariable calculating process S21, the fitted curve determining process S22 is performed with respect to every vulcanizing temperature.
Fig. 8 shows a flowchart of the fitted curve determining process S22 in this embodiment.

*** process S221

The process S221 is performed with respect to one of the vulcanizing temperatures.
In this process S221, with respect to each of the plural sets of values of the invariables K1, K2 and m, by assigning the values of K1, K2 and m to the expression (1), an estimated vulcanization curve 3 of the expression (1) is obtained. Therefore, a plurality of the estimated vulcanization curves 3 are obtained as shown in Figs.2-5.
In Figs.2-5, the estimated vulcanization curves 3 of only three of the real numbers are shown.

*** process S222

The process S222 is performed with respect to the vulcanizing temperature concerned.
In this process S222, from a plurality of the estimated vulcanization curves 3, an estimated vulcanization curve 3 whose error from the measured vulcanization curve 2 is smallest, namely, most fitted curve 3s is determined or found out. The error between the estimated vulcanization curve 3 and the measured vulcanization curve 2 can be obtained by summing up the difference X1(t)-X2(t) between
the vulcanization degree X1(t) of the estimated vulcanization curve 3 at the vulcanizing time t and
the vulcanization degree X2(t) of the measured vulcanization curve 2 at the vulcanizing time t from the vulcanizing time t when the vulcanization degree X of the measured vulcanization curve 2 is 0 to the vulcanizing time t when the vulcanization degree X of the measured vulcanization curve 2 is 1.

*** process S223

In the process S223, it is judged if the most fitted curve 3s has been determined with respect to every vulcanizing temperature for example, 150, 160, 170 and 180 degrees C. If "yes", then the process S23 goes to process S23.
If "no" namely there exists a vulcanizing temperature about which the most fitted curve 3s is not yet determined, then with respect to that vulcanizing temperature, the process S221 to S222 are repeated.
Therefore, in the fitted curve determining process S22, the most fitted curves 3s are obtained with respect to all of the vulcanizing temperatures as shown in Fig. 2 to Fig. 5.

** process S23

In the process S23, firstly, the values of invariable K1 of the most fitted curves 3s and their vulcanizing temperatures are plotted as shown in Fig. 9(a).
Then, an approximate straight line to the plot points is determined, and the linear expression of such line, namely, approximate expression K1(s) is obtained.
Thus, by using the approximate expression K1(s), it is possible to obtain the value of the invariable K1 at any vulcanizing temperature s.
For example, the approximate expression is

$$K1(s)=-2.17\times10^{-4} + 1.67\times10^{-6} \times s.$$

** process S24

In the process S24, firstly, the values of invariable K2 of the most fitted curves 3s and their vulcanizing temperatures are plotted as shown in Fig. 9(b).
Then, an approximate straight line to the plot points is determined, and the linear expression of such line, namely, approximate expression K2(s) is obtained.
Thus, by using the approximate expression K2(s), it is possible to obtain the value of the invariable K2 at any vulcanizing temperature s.
For example, the approximate expression is

$$K2(s)=-2.17X10^{-4} + 2.24X10^{-3} X s.$$

** process S25

In the process S25, firstly, the values of invariable m of the most fitted curves 3s and their vulcanizing temperatures are plotted as shown in Fig. 9(c).
Then, an approximate straight line to the plot points is determined, and the linear expression of such line, namely, approximate expression m(s) is obtained.
Thus, by using the approximate expression m(s), it is possible to obtain the value of the invariable m at any vulcanizing temperature s.
For example, the approximate expression is

$$m(s)=4.14X10^{-1} + 3.01X10^{-3} X s.$$

Accordingly, by using the approximate expressions K1(s), K2(s) and m(s), the values of the invariables K1, K2 and m can be determined with respect to any vulcanizing temperature s.
Therefore, by assigning the determined values of the invariables K1, K2 and m to the expression (1), the most fitted curve 3s (estimated vulcanization curve 3) at any vulcanizing temperature s can be defined easily in a short time in order to accurately estimate the vulcanization degree of the rubber compound.

* Estimating process S3

Fig. 10 shows an example of the change curve 7 of the vulcanizing temperature s with the vulcanizing time t as an examination object to be examined as to whether the desired vulcanization degree can be achieved in a process of vulcanizing a rubber compound.
In the estimating process S3 in this embodiment, with respect to such change curve 7, the change in the vulcanization degree X(t) of the rubber compound with the vulcanizing time t is estimated.
Fig. 11 shows a flowchart of the estimating process S3.

** process S31

In the process S31, first, the vulcanizing temperature s corresponding to a certain point in the vulcanizing time t is obtained from the change curve 7 as shown in Fig. 10. Initially, starting time t1 (=0) is used as a certain point in the vulcanizing time t.
Then, the obtained vulcanizing temperature s is assigned to the above-mentioned approximate expressions K1(t), K2(t) and m(t), and the values of the invariables K1, K2 and m are obtained therefrom.

** process S32

In the process S32, the obtained values of the invariables K1, K2 and m are assigned to the expression (1), and the gradient (dx/dt) of the vulcanization degree at the certain point in the vulcanizing time t is obtained.

** process S33

In the process S33, the obtained gradient (dx/dt) is assigned to the following expression (2)

$$X(t+1) = X(t) + \frac{dX}{dt} X(t) \qquad (t > 0)$$
$$X(0) = 0$$

Expression (2).

Thereby, the vulcanization degree X(t+1) at the incremented vulcanizing time t+1 is calculated.

** process S34

In the process S34, it is judged if the vulcanization degree X(t) has been calculated throughout the entire range of the vulcanizing time t.
If "yes" namely the vulcanizing time t has reached to the termination time of the vulcanization, then the procedure goes to process S36.
If "no" namely the vulcanizing time t is less than the termination time of the vulcanization, then the procedure goes to process S35.

** process S35

In the process S35, the vulcanizing time t is incremented.
Then, the process S31 to S34 are repeated.
Thus, in the estimating process S3, from the beginning to the end of the vulcanization shown in Fig. 10, the vulcanization degree X(t) can be estimated from moment to moment.

** process S36

In the process S36, as shown in Fig. 12, the obtained vulcanization degree X(t) and the vulcanizing time t are plotted as an estimated vulcanization curve 5.

** process S37

Next, in the process S37, it is judged if the change in the vulcanization degree X(t) with the vulcanizing time is desirable.
If "yes", then the procedure goes to process S38.
If "no", then the procedure goes to process S39.

** process S38

In the process S38, the rubber product is manufactured by vulcanizing a rubber compound according to the change curve 7 as shown in Fig. 10.

** process S39

In the process S39, the change curve 7 is modified.
Then, the process S31 to S37 are again performed.
In the case of a pneumatic tire including plural kinds of rubber members, e.g. tread rubber, sidewall rubber and the like, it is desirable that the time t required for the vulcanization degree X(t) of each rubber member becoming within a range of from 0.8 to 1.0 is not widely varied among the rubber members, in other words, the times t are within a specific range. In order to examine this, the above described method in this embodiment can be suitably used.
For example, the vulcanizing time of a tire is determined by the longest one of the times required for the vulcanization degree X(t) of the rubber members becoming 0.8. In order to find an optimal condition which can reduce the vulcanizing time, the above described method in this embodiment can be suitably used.

[Embodiment 1]

According to the procedure shown in Fig. 1, for each of the vulcanizing temperatures, 150, 160, 170 and 180 degrees C, the measured vulcanization curve of the rubber compound was obtained.
For each of the vulcanizing temperatures, the values of invariables K1, K2 and m, which minimize the error between the measured vulcanization curve and the estimated vulcanization curve defined by of the expression (1), were obtained. Then, the error (maximum) between the vulcanization degree x(t) of the estimated vulcanization curve obtained by assigning the values of invariables K1, K2 and m and
the vulcanization degree X(t) of the measured vulcanization curve was calculated, The results are as follows.
Error at 150 degrees C: 0.08
Error at 160 degrees C: 0.08
Error at 170 degrees C: 0.08
Error at 180 degrees C: 0.08
If the error is less than 0.1 at any point in the vulcanizing time t, the estimated vulcanization curve is considered as being good.
In the embodiment 1, therefore, accurate estimated vulcanization curves could be obtained. In other words, the vulcanization degree of the rubber compound at any point in the vulcanizing time t could be accurately estimated.

[Embodiment 2]

while vulcanizing the rubber compound according to the change curve 7 shown in Fig. 10, the vulcanization degree or torque was measured according to JIS K 6300, and the change in the vulcanization degree with the vulcanizing time was obtained.
According to the procedure shown in Fig. 1 and the procedure (S31-S36) shown in Fig. 11, an estimated vulcanization curve 5 as shown in Fig. 12 corresponding to the change curve 7 shown in Fig. 10 was obtained.
The vulcanizing temperatures were same as those in the embodiment 1. The plural sets of the values for the invariables K1, K2 and m obtained in the embodiment 1 were used. Then, the error (maximum) between the estimated vulcanization curve 5 and the measured change in the vulcanization degree with the vulcanizing time was calculated.
As a result, the maximum error was 0.07.
If the error is less than 0.1 at any point in the vulcanizing time t, the estimated vulcanization curve is considered as being good.
Therefore, the vulcanization degree of the rubber compound at any point in the vulcanizing time t could be accurately estimated.

## Claims

1. A method for estimating the vulcanization degree of a rubber compound, comprising
a measuring process (S1) in which a measured vulcanization curve (2) which is a change curve of the vulcanization degree of the rubber compound with vulcanizing time is obtained by actually measuring the shearing resistance (or torque) of the rubber compound during vulcanization,
an invariable determining process (S2) in which values of the invariables K1, K2 and m of the following expression (1) are determined which values minimize the error between the measured vulcanization curve (2) and an estimated vulcanization curve (3) which is a curve of the expression (1),

$$\ln\left[\frac{1}{(1-X)}\frac{dX}{dt}-K_1\right]=\ln(K_2)+m\ln(X) \qquad \text{Expression (1)}$$

wherein

x: vulcanization degree
t: vulcanizing time

wherein
the invariable determining process (S2) comprises
an invariable calculating process (S21) in which each of different real numbers is assigned to the invariable K1 of the expression (1), wherein the real numbers are more than 0 and less than 1, and
a linear expression (4), whose gradient and intercept are equal to m and K2 respectively, is transformed from the expression (1) and is approximated to the measured vulcanization curve (2), and then from the approximated expression, a set of values of the invariables K1, K2 and m are obtained,
whereby plural sets of the values corresponding to the respective real numbers are obtained, and

a fitted curve determining process (S22) in which a plurality of the estimated vulcanization curves (3) are obtained by assigning the plural sets of the values to the expression (1), and
from a plurality of the estimated vulcanization curves (3), an estimated vulcanization curve 3s whose error from the measured vulcanization curve (2) is smallest, is found out.

**2.** The method for estimating the vulcanization degree of a rubber compound according to claim 1, wherein
in the measuring process (S1), the measured vulcanization curve (2) is obtained with respect to each of different vulcanizing temperatures, and
in the fitted curve determining process (S22), for each of the different vulcanizing temperatures, the estimated vulcanization curve (3s) whose error from the measured vulcanization curve is smallest is obtained.

**3.** The method for estimating the vulcanization degree of a rubber compound according to claim 2, wherein
the invariable determining process (S2) comprises
a process (S23) in which a relation between the values of invariable K1 of the estimated vulcanization curves obtained in the fitted curve determining process (S22) and their vulcanizing temperatures is approximated by a straight line, and the linear expression (4) of the straight line is obtained as an approximate expression of the invariable K1 in relation to the vulcanizing temperature,
a process (S24) in which a relation between the values of invariable K2 of the estimated vulcanization curves obtained in the fitted curve determining process (S22) and their vulcanizing temperatures is approximated by a straight line, and the linear expression (4) of the straight line is obtained as an approximate expression of the invariable K2 in relation to the vulcanizing temperature, and
a process (S25) in which a relation between the values of invariable m of the estimated vulcanization curves obtained in the fitted curve determining process (S22) and their vulcanizing temperatures is approximated by a straight line, and the linear expression (4) of the straight line is obtained as an approximate expression of the invariable m in relation to the vulcanizing temperature.

**4.** The method for estimating the vulcanization degree of a rubber compound according to claim 3, which comprises an estimating process (S3) in which,
for a first change curve of a vulcanizing temperature with vulcanizing time designed to vulcanize the rubber compound, a second change curve of the vulcanization degree X of the rubber compound with the vulcanizing time is estimated,
the estimating process (S3) comprises
a process in which the vulcanizing temperature corresponding to a certain point in the vulcanizing time in the first change curve (7) is assigned to
the approximate expression of the invariable K1,
the approximate expression of the invariable K2 and
the approximate expression of the invariable m, and
values of the invariables K1, K2 and m corresponding to the certain point in the vulcanizing time are obtained,
a process (S32) in which the obtained values of the invariables K1, K2 and m corresponding to the certain point in the vulcanizing time are assigned to the expression (1) and the gradient (dx/dt) of the vulcanization degree at the certain point in the vulcanizing time is obtained,
a process (S33) in which, by assigning the obtained gradient (dx/dt) to the following expression (2) and using the vulcanization degree X(t) at the certain point in the vulcanizing time, the vulcanization degree X(t+1) after a micro time dt is calculated,

$$X(t+1) = X(t) + \frac{dX}{dt} X(t) \qquad (t > 0)$$
$$X(0) = 0$$

Expression (2).

**Patentansprüche**

**1.** Verfahren zur Schätzung des Vulkanisationsgrades einer Kautschukmischung, umfassend

einen Messprozess (S1), in dem eine gemessene Vulkanisationskurve (2), welche eine Änderungskurve des Vulkanisationsgrades der Kautschukmischung über die Vulkanisationszeit ist, dadurch erhalten wird, dass der Scherwiderstand (oder das Drehmoment) der Kautschukmischung während der Vulkanisation tatsächlich gemessen wird,

einen Konstantenbestimmungsprozess (S2), in dem Werte der Konstanten K1, K2 und m des folgenden Ausdrucks (1) bestimmt werden, welche die Abweichung zwischen der gemessenen Vulkanisationskurve (2) und einer geschätzten Vulkanisationskurve (3) minimieren, die eine Kurve des Ausdrucks (1) ist,

$$\ln\left[\frac{1}{(1-X)}\frac{dX}{dt}-K_1\right]=\ln(K_2)+m\ln(X) \qquad \text{Ausdruck (1)}$$

wobei

X: Vulkanisationsgrad

t: Vulkanisationszeit

wobei

der Konstantenbestimmungsprozess (S2) umfasst:

einen Konstantenberechnungsprozess (S21), in welchem jede von verschiedenen reellen Zahlen der Konstanten K1 des Ausdrucks (1) zugeordnet wird, wobei die reellen Zahlen größer als 0 und kleiner als 1 sind, und ein linearer Ausdruck (4), dessen Gradient und Achsenabschnitt jeweils gleich m und K2 sind, aus dem Ausdruck (1) transformiert und an die gemessene Vulkanisationskurve (2) angenähert wird, und dann aus dem angenäherten Ausdruck ein Satz von Werten für die Konstanten K1, K2 und m erhalten wird,

wodurch mehrere Sätze von Werten erhalten werden, die den jeweiligen reellen Zahlen entsprechen, und

einen Bestimmungsprozess für eine angepasste Kurve (S22), in welchem eine Mehrzahl geschätzter Vulkanisationskurven (3) erhalten wird, indem die mehreren Sätze von Werten dem Ausdruck (1) zugeordnet werden, und

aus einer Mehrzahl der geschätzten Vulkanisationskurven (3) eine geschätzte Vulkanisationskurve 3s ermittelt wird, deren Abweichung von der gemessenen Vulkanisationskurve (2) am geringsten ist.

**2.** Verfahren zur Schätzung des Vulkanisationsgrades einer Kautschukmischung gemäß Anspruch 1, wobei

im Messprozess (S1) die gemessene Vulkanisationskurve (2) hinsichtlich jeder von verschiedenen Vulkanisationstemperaturen erhalten wird, und

im Bestimmungsprozess für eine angepasste Kurve (S22) für jede der verschiedenen Vulkanisationstemperaturen die geschätzte Vulkanisationskurve (3s) erhalten wird, deren Abweichung von der gemessenen Vulkanisationskurve am geringsten ist.

**3.** Verfahren zur Schätzung des Vulkanisationsgrades einer Kautschukmischung gemäß Anspruch 2, wobei der Konstantenbestimmungsprozess (S2) umfasst:

einen Prozess (S23), in welchem eine Relation zwischen den Werten der Konstanten K1 der geschätzten Vulkanisationskurven, die im Bestimmungsprozess für eine angepasste Kurve (S22) erhalten wurden, und ihren Vulkanisationstemperaturen durch eine Gerade angenähert wird, und der lineare Ausdruck (4) der Geraden als ein näherungsweiser Ausdruck der Konstanten K1 in Relation zur Vulkanisationstemperatur erhalten wird,

einen Prozess (S24), in welchem eine Relation zwischen den Werten der Konstanten K2 der geschätzten Vulkanisationskurven, die im Bestimmungsprozess für eine angepasste Kurve (S22) erhalten wurden, und ihren Vulkanisationstemperaturen durch eine Gerade angenähert wird und der lineare Ausdruck (4) der Geraden als ein näherungsweiser Ausdruck der Konstanten K2 in Relation zur Vulkanisationstemperatur erhalten wird, und

einen Prozess (S25), in welchem eine Relation zwischen den Werten der Konstanten m der geschätzten Vulkanisationskurven, die im Bestimmungsprozess für eine angepasste Kurve (S22) erhalten wurden, und ihren Vulkanisationstemperaturen durch eine Gerade angenähert wird und der lineare Ausdruck (4) der Geraden als ein näherungsweiser Ausdruck der Konstanten m in Relation zur Vulkanisationstemperatur erhalten wird.

**4.** Verfahren zur Schätzung des Vulkanisationsgrades einer Kautschukmischung gemäß Anspruch 3, welches

einen Schätzungsprozess (S3) umfasst, in welchem für eine erste Änderungskurve einer Vulkanisationstemperatur über eine Vulkanisationszeit, die dazu ausgelegt ist, die Kautschukmischung zu vulkanisieren, eine zweite Änderungskurve des Vulkanisationsgrades X der Kautschukmischung über die Vulkanisationszeit geschätzt wird,
wobei der Schätzungsprozess (S3) umfasst:

einen Prozess, in welchem die Vulkanisationstemperatur, die einem bestimmten Punkt in der Vulkanisationszeit in der ersten Änderungskurve (7) entspricht, dem näherungsweisen Ausdruck der Konstanten K1, dem näherungsweisen Ausdruck der Konstanten K2 und dem näherungsweisen Ausdruck der Konstanten m zugeordnet wird, und
Werte der Konstanten K1, K2 und m erhalten werden, die dem bestimmten Punkt in der Vulkanisationszeit entsprechen,
einen Prozess (S32), in welchem die erhaltenen Werte der Konstanten K1, K2 und m, die dem bestimmten Punkt in der Vulkanisationszeit entsprechen, dem Ausdruck (1) zugeordnet werden und der Gradient (dX/dt) des Vulkanisationsgrades an dem bestimmten Punkt in der Vulkanisationszeit erhalten wird, und
einen Prozess (S33), in welchem durch Zuordnung des erhaltenen Gradienten (dX/dt) zum folgenden Ausdruck (2) und unter Verwendung des Vulkanisationsgrades X(t) an dem bestimmten Punkt in der Vulkanisationszeit der Vulkanisationsgrad X(t+1) nach einer Mikrozeit dt berechnet wird,

$$X(t+1) = X(t) + \frac{dX}{dt} X(t) \qquad (t > 0)$$
$$X(0) = 0$$
Ausdruck (2).

**Revendications**

1. Procédé pour estimer le degré de vulcanisation d'un composé de caoutchouc, comprenant
un processus de mesure (S1) dans lequel une courbe de vulcanisation mesurée (2) qui est une courbe de changement du degré de vulcanisation du composé de caoutchouc en fonction du temps de vulcanisation est obtenue en mesurant réellement la résistance au cisaillement (ou le couple) du composé de caoutchouc pendant la vulcanisation,
un processus de détermination d'invariables (S2) dans lequel des valeurs des invariables K1, K2 et m de l'expression suivante (1) sont déterminées, lesdites valeurs minimisant l'erreur entre la courbe de vulcanisation mesurée (2) et une courbe de vulcanisation estimée (3) qui est une courbe de l'expression (1),

$$\ln\left[\frac{1}{(1-X)}\frac{dX}{dt} - K_1\right] = \ln(K_2) + m\ \ln(X) \qquad \text{Expression (1)}$$

dans laquelle

X est le degré de vulcanisation
t est le temps de vulcanisation

dans lequel le processus de détermination d'invariables (S2) comprend un processus de calcul d'invariables (S21) dans lequel chacun parmi des différents nombres réels est attribué à l'invariable K1 de l'expression (1), dans lequel les nombres réels sont supérieurs à 0 et inférieurs à 1, et
une expression linéaire (4), dont le gradient et l'interception sont égaux à m et K2 respectivement, est transformée à partir de l'expression (1) et est approximée à la courbe de vulcanisation mesurée (2), et ensuite
à partir de l'expression approximée, on obtient un groupe de valeurs des invariables K1, K2 et m,
de sorte que l'on obtient une pluralité de groupes de valeurs correspondants aux nombres réels respectifs, et
un processus de détermination de courbe ajustée (S22) dans lequel une pluralité de courbes de vulcanisation estimées (3) sont obtenues en attribuant la pluralité de groupes des valeurs à l'expression (1), et
à partir d'une pluralité de courbes de vulcanisation estimées (3), on retrouve une courbe de vulcanisation estimée (3s) dont l'erreur par rapport à la courbe de vulcanisation mesurée (2) est la plus petite.

2. Procédé pour estimer le degré de vulcanisation d'un composé de caoutchouc selon la revendication 1, dans lequel dans le processus de mesure (S1), la courbe de vulcanisation mesurée (2) est obtenue à l'égard de chacune de différentes températures de vulcanisation, et

dans le processus de détermination de courbe ajustée (S22), pour chacune des différentes températures de vulcanisation, on obtient la courbe de vulcanisation estimée (3s) dont l'erreur par rapport à la courbe de vulcanisation mesurée est la plus petite.

**3.** Procédé pour estimer le degré de vulcanisation d'un composé de caoutchouc selon la revendication 2, dans lequel le processus de détermination d'invariables (S2) comprend
un processus (S23) dans lequel une relation entre les valeurs de l'invariable K1 des courbes de vulcanisation estimées obtenues dans le processus de détermination de courbe ajustée (S22) et leurs températures de vulcanisation est approximée par une ligne droite, et l'expression linéaire (4) de la ligne droite est obtenue comme une expression approximative de l'invariable K1 en relation à la température de vulcanisation,
un processus (S24) dans lequel une relation entre les valeurs de l'invariable K2 des courbes de vulcanisation estimées obtenues dans le processus de détermination de courbe ajustée (S22) et leurs températures de vulcanisation est approximée par une ligne droite, et l'expression linéaire (4) de la ligne droite est obtenue comme une expression approximative de l'invariable K2 en relation à la température de vulcanisation, et
un processus (S25) dans lequel une relation entre les valeurs de l'invariable m des courbes de vulcanisation estimées obtenues dans le processus de détermination de courbe ajustée (S22) et leurs températures de vulcanisation est approximée par une ligne droite, et l'expression linéaire (4) de la ligne droite est obtenue à titre d'expression approximative de l'invariable m en relation à la température de vulcanisation.

**4.** Procédé pour estimer le degré de vulcanisation d'un composé de caoutchouc selon la revendication 3, qui comprend un processus d'estimation (S3) dans lequel,
pour une première courbe de changement d'une température de vulcanisation avec un temps de vulcanisation conçu pour vulcaniser le composé de caoutchouc, une seconde courbe de changement du degré de vulcanisation X du composé de caoutchouc avec le temps de vulcanisation est estimée,
le processus d'estimation (S3) comprend
un processus dans lequel la température de vulcanisation correspondant à un certain point dans le temps de vulcanisation dans la première courbe de changement (7) est attribuée à l'expression approximative de l'invariable K1, de l'expression approximative de l'invariable K2, et de l'expression approximative de l'invariable m, et des valeurs des invariables K1, K2 et m correspondant au certain point dans le temps de vulcanisation sont obtenues,
un processus (S32) dans lequel les valeurs obtenues des invariables K1, K2 et m correspondant au certain point dans le temps de vulcanisation sont attribuées à l'expression (1) et le gradient (dX/dt) du degré de vulcanisation au certain point dans le temps de vulcanisation est obtenu,
un processus (S33) dans lequel, en attribuant le gradient obtenu (dX/dt) à l'expression suivante (2) et en utilisant le degré de vulcanisation X(t) au certain point dans le temps de vulcanisation, le degré de vulcanisation X(t+1) après un micro temps dt est calculé,

$$X(t+1) = X(t) + \frac{dX}{dt} X(t) \qquad (t > 0)$$

$$X(0) = 0$$

Expression (2).

FIG.1

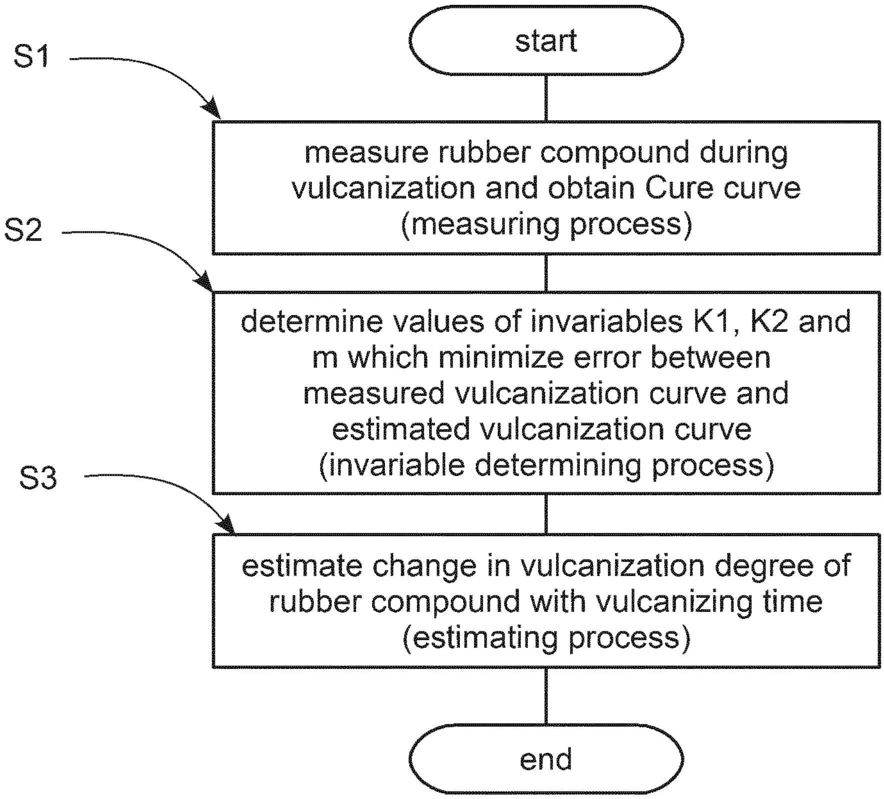
start
S1
measure rubber compound during vulcanization and obtain Cure curve (measuring process)
S2
determine values of invariables K1, K2 and m which minimize error between measured vulcanization curve and estimated vulcanization curve (invariable determining process)
S3
estimate change in vulcanization degree of rubber compound with vulcanizing time (estimating process)
end

FIG.2

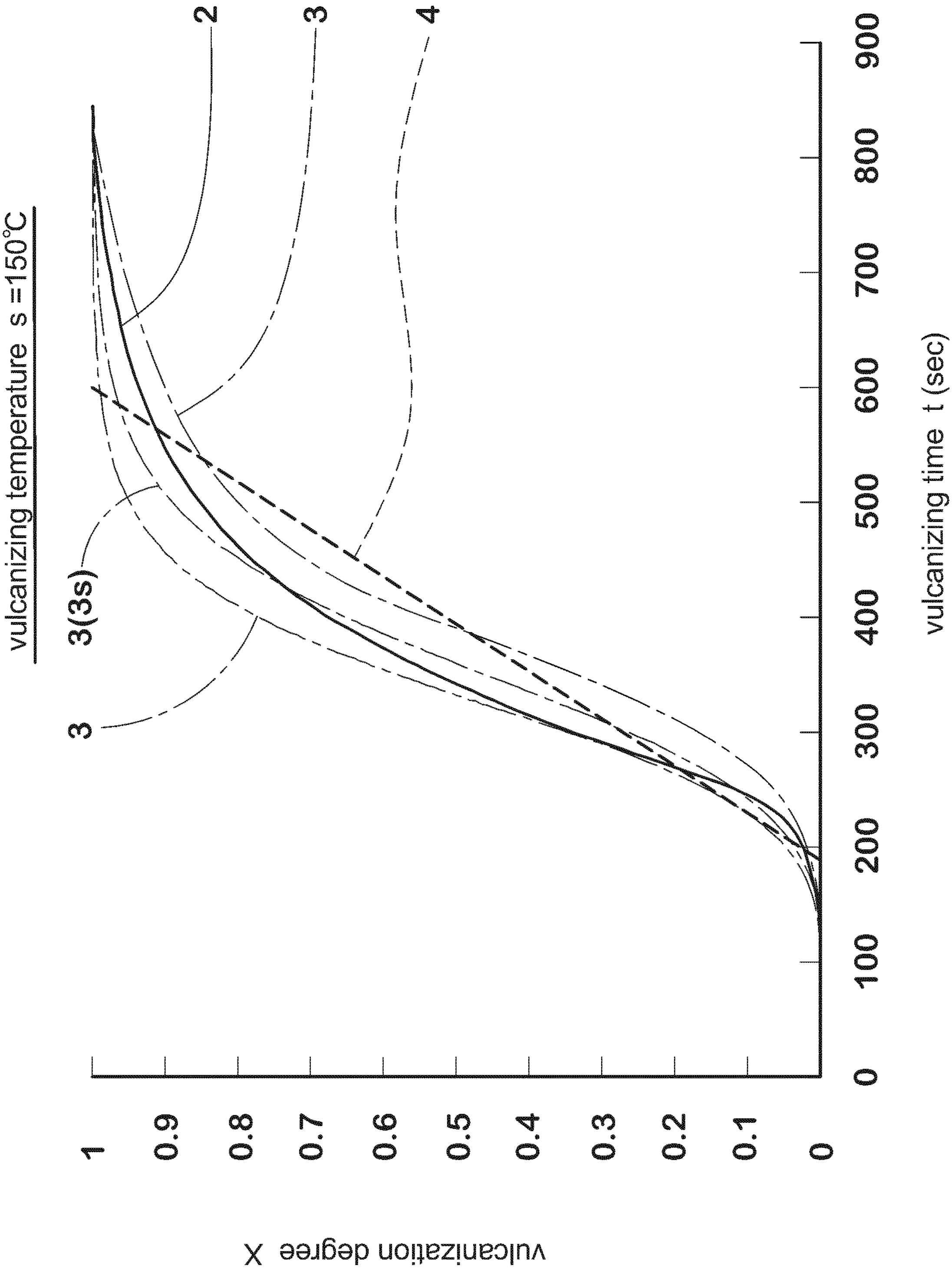
vulcanizing temperature s =150°C
2
3
4
3
3(3s)
vulcanization degree X
vulcanizing time t (sec)
0
0.1
0.2
0.3
0.4
0.5
0.6
0.7
0.8
0.9
1
0
100
200
300
400
500
600
700
800
900

FIG.3

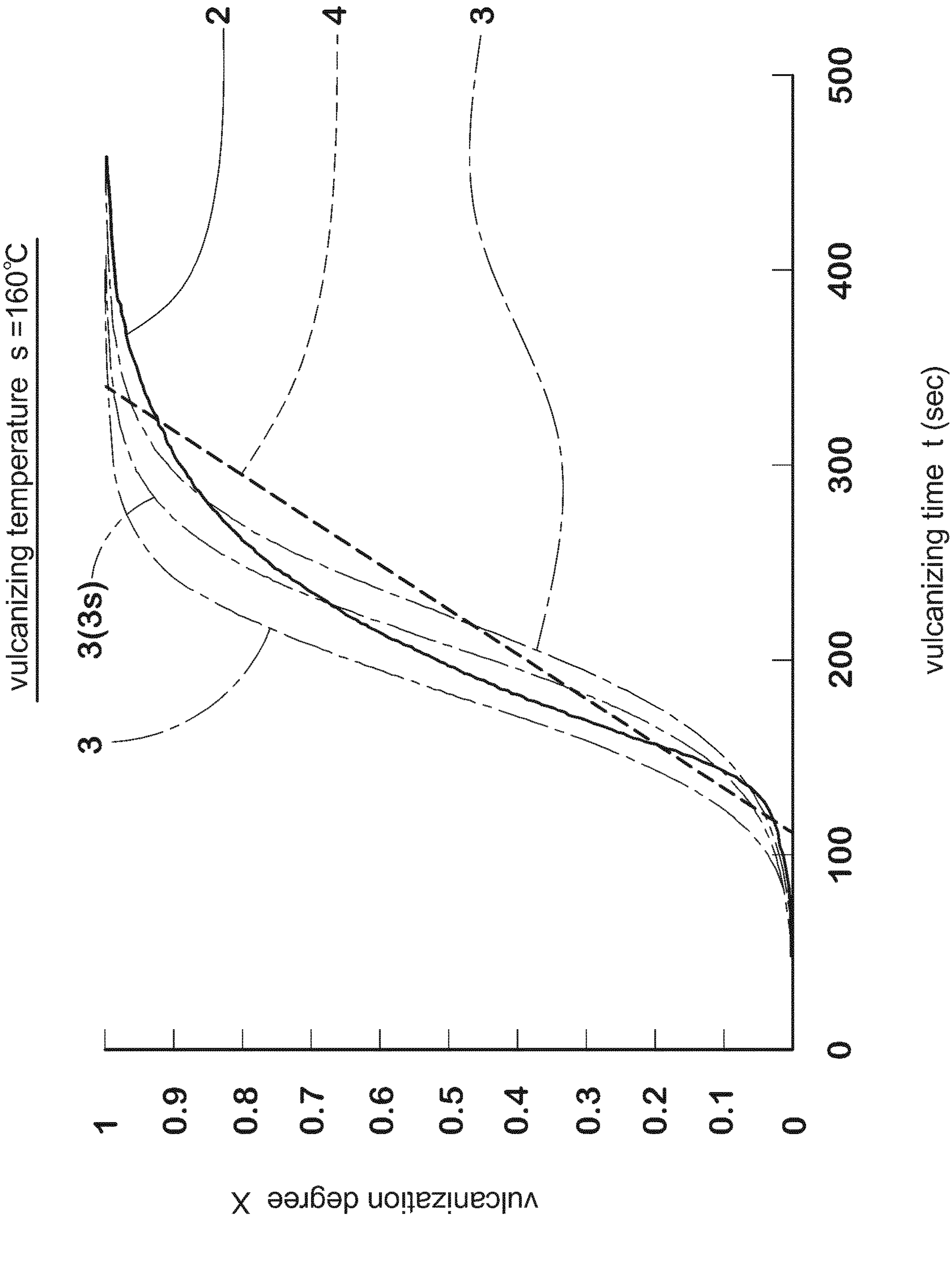
vulcanizing temperature s =160°C
vulcanization degree X
1
0.9
0.8
0.7
0.6
0.5
0.4
0.3
0.2
0.1
0
vulcanizing time t (sec)
0
100
200
300
400
500
3
3(3s)
2
4
3

FIG.4

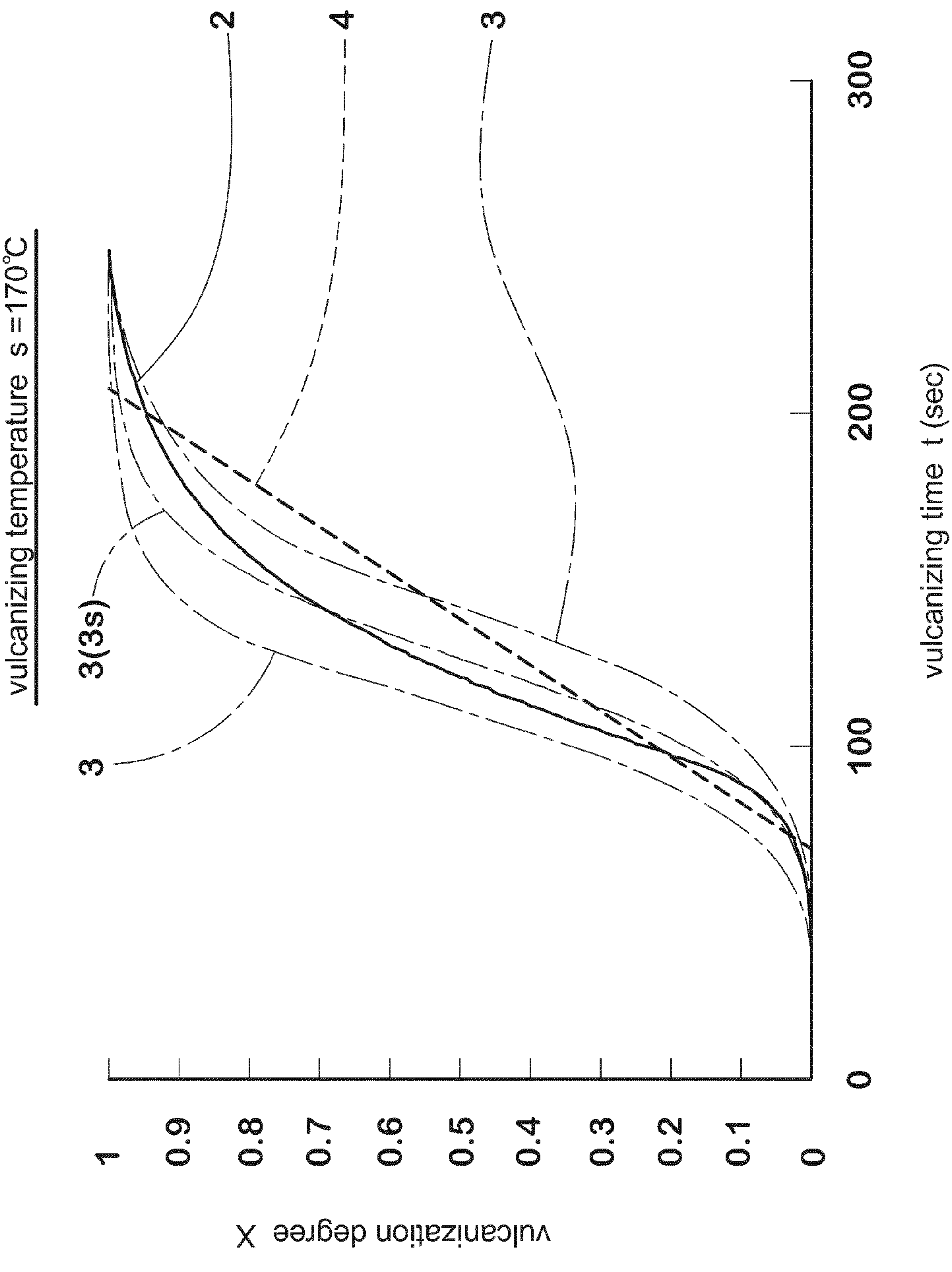
vulcanizing temperature s =170°C
vulcanization degree X
vulcanizing time t (sec)
0
0.1
0.2
0.3
0.4
0.5
0.6
0.7
0.8
0.9
1
0
100
200
300
2
4
3
3(3s)
3

FIG.5

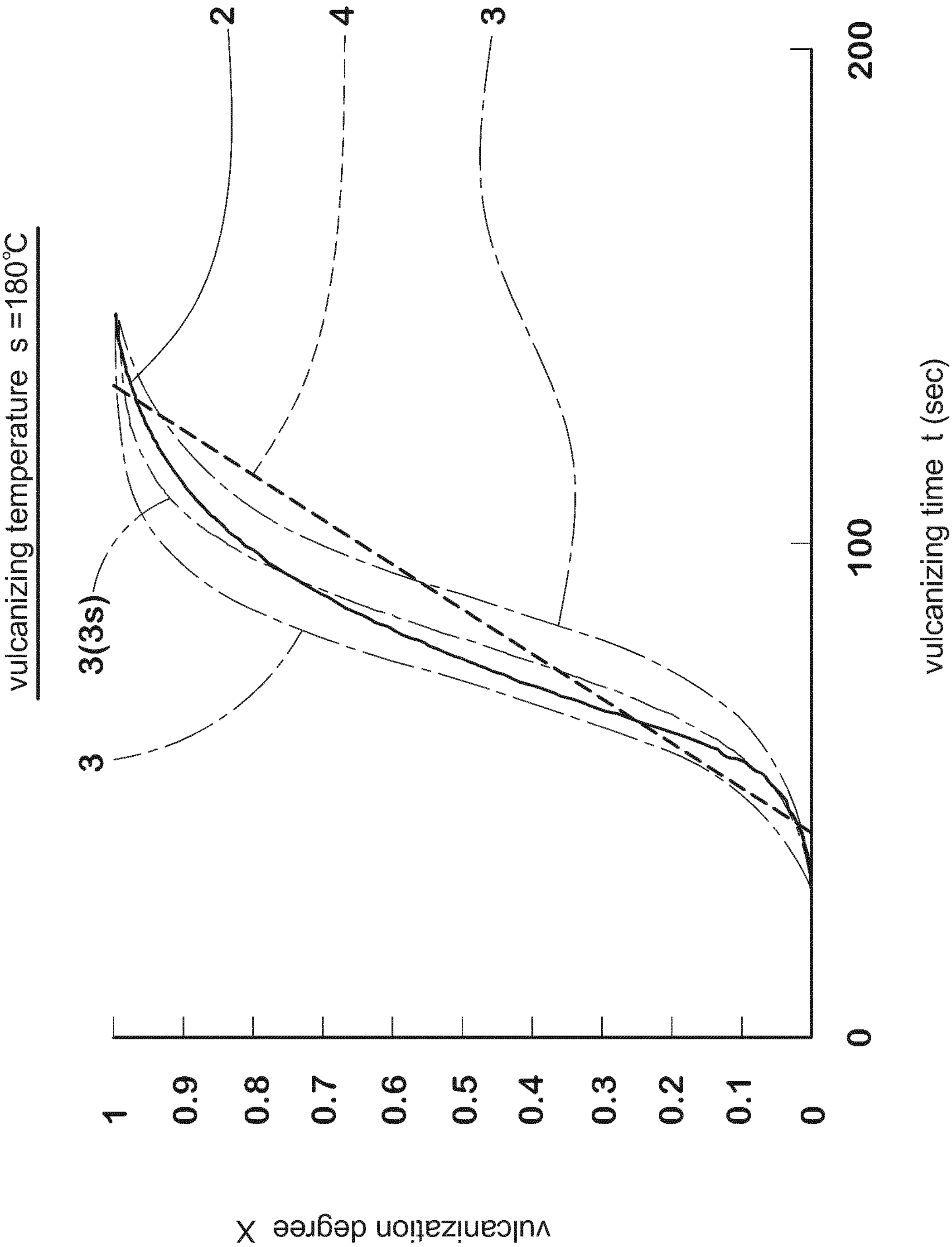
vulcanizing temperature s =180°C
vulcanization degree X
1
0.9
0.8
0.7
0.6
0.5
0.4
0.3
0.2
0.1
0
0
100
200
vulcanizing time t (sec)
2
4
3
3(3s)
3

FIG.6

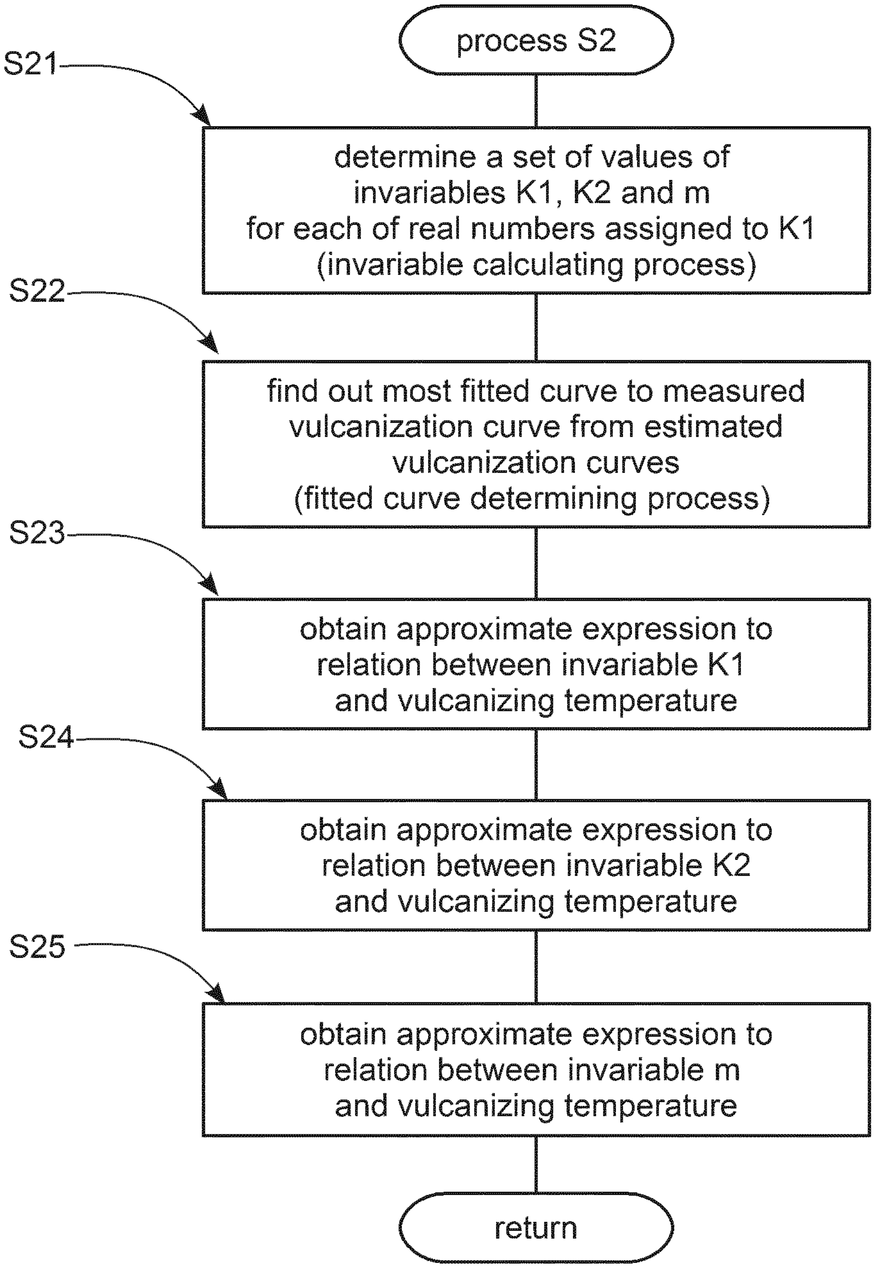
process S2
S21
determine a set of values of invariables K1, K2 and m for each of real numbers assigned to K1 (invariable calculating process)
S22
find out most fitted curve to measured vulcanization curve from estimated vulcanization curves (fitted curve determining process)
S23
obtain approximate expression to relation between invariable K1 and vulcanizing temperature
S24
obtain approximate expression to relation between invariable K2 and vulcanizing temperature
S25
obtain approximate expression to relation between invariable m and vulcanizing temperature
return

FIG.7

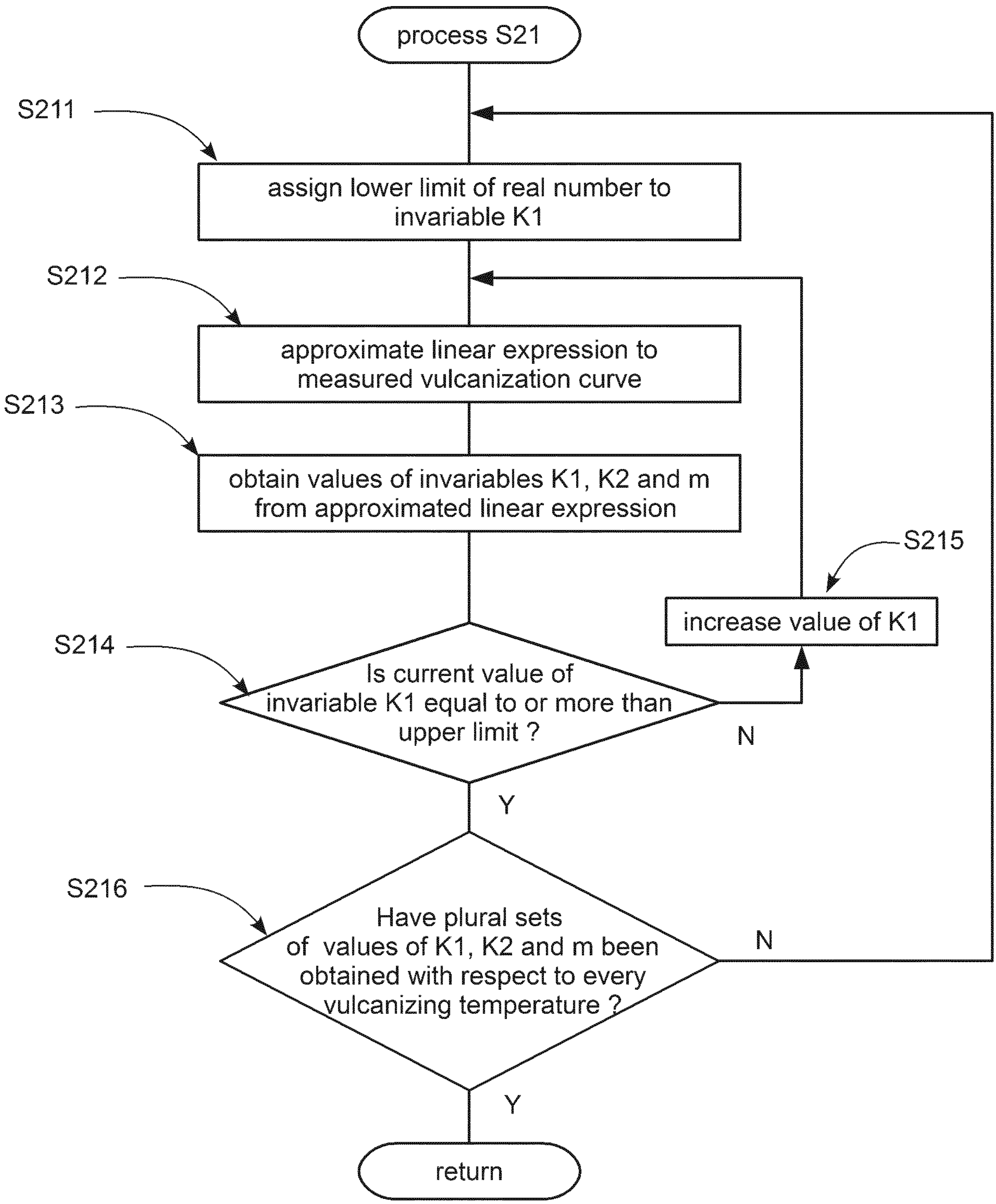
process S21
S211
assign lower limit of real number to invariable K1
S212
approximate linear expression to measured vulcanization curve
S213
obtain values of invariables K1, K2 and m from approximated linear expression
S215
increase value of K1
S214
Is current value of invariable K1 equal to or more than upper limit ?
N
Y
S216
Have plural sets of values of K1, K2 and m been obtained with respect to every vulcanizing temperature ?
N
Y
return

FIG.8

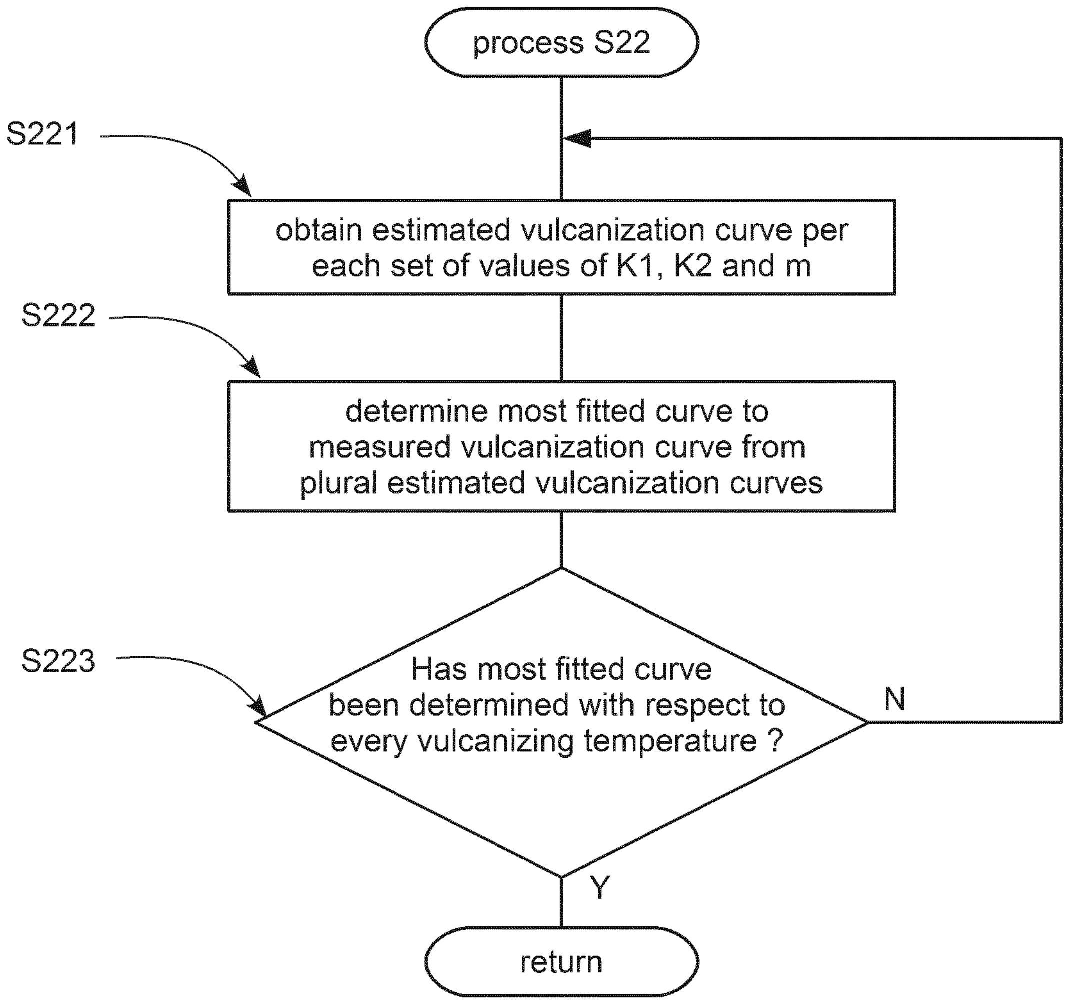
process S22
S221
obtain estimated vulcanization curve per each set of values of K1, K2 and m
S222
determine most fitted curve to measured vulcanization curve from plural estimated vulcanization curves
S223
Has most fitted curve been determined with respect to every vulcanizing temperature ?
N
Y
return

FIG.9(a)

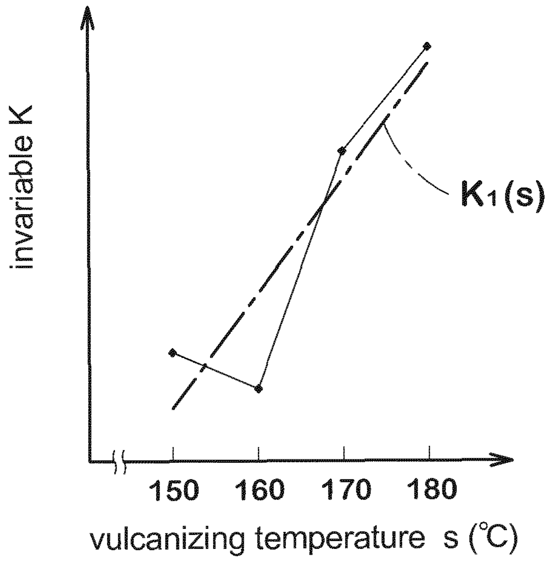


FIG.9(b)

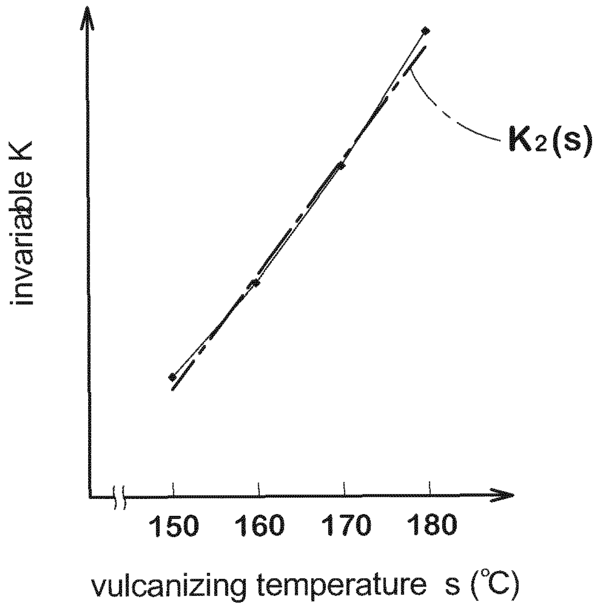


FIG.9(c)

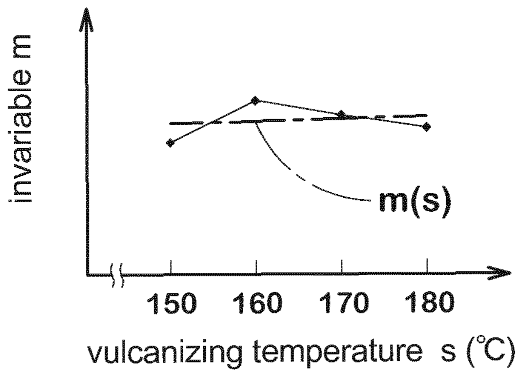

FIG.10

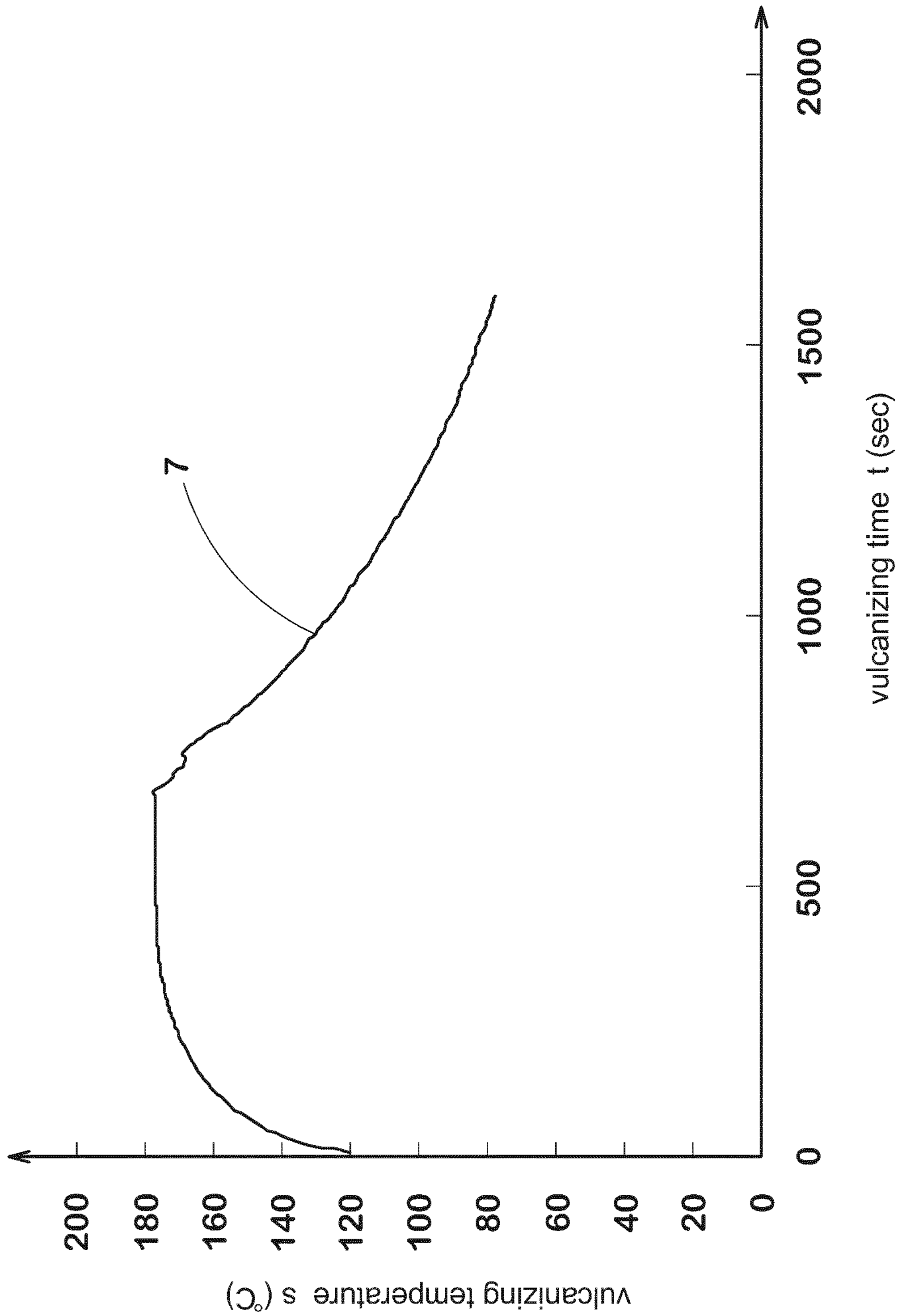
vulcanizing temperature s (°C)
200
180
160
140
120
100
80
60
40
20
0
0
500
1000
1500
2000
vulcanizing time t (sec)
7

FIG.11

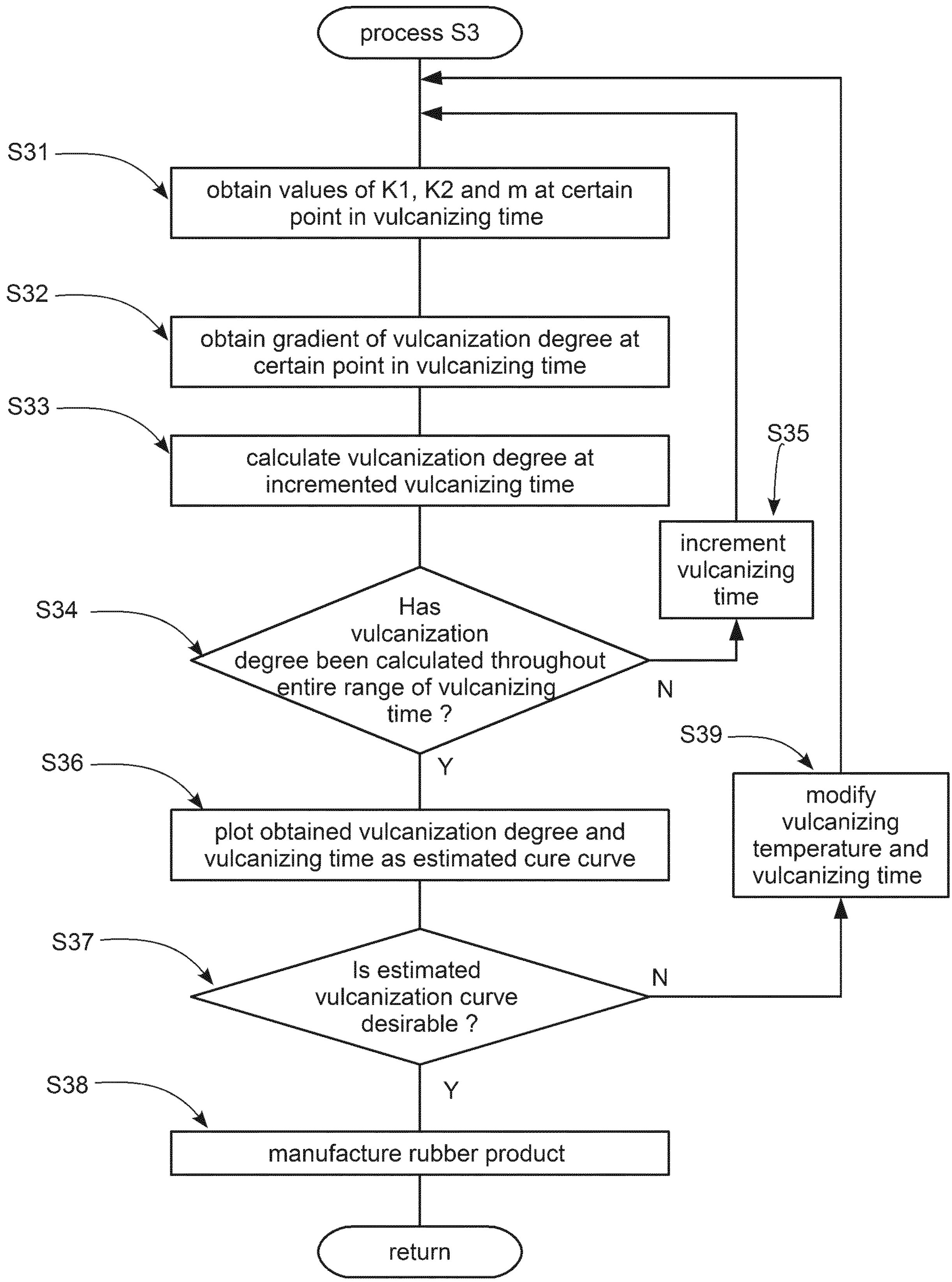
process S3
S31
obtain values of K1, K2 and m at certain point in vulcanizing time
S32
obtain gradient of vulcanization degree at certain point in vulcanizing time
S33
calculate vulcanization degree at incremented vulcanizing time
S34
Has vulcanization degree been calculated throughout entire range of vulcanizing time ?
N
S35
increment vulcanizing time
Y
S36
plot obtained vulcanization degree and vulcanizing time as estimated cure curve
S37
Is estimated vulcanization curve desirable ?
N
S39
modify vulcanizing temperature and vulcanizing time
Y
S38
manufacture rubber product
return

FIG.12

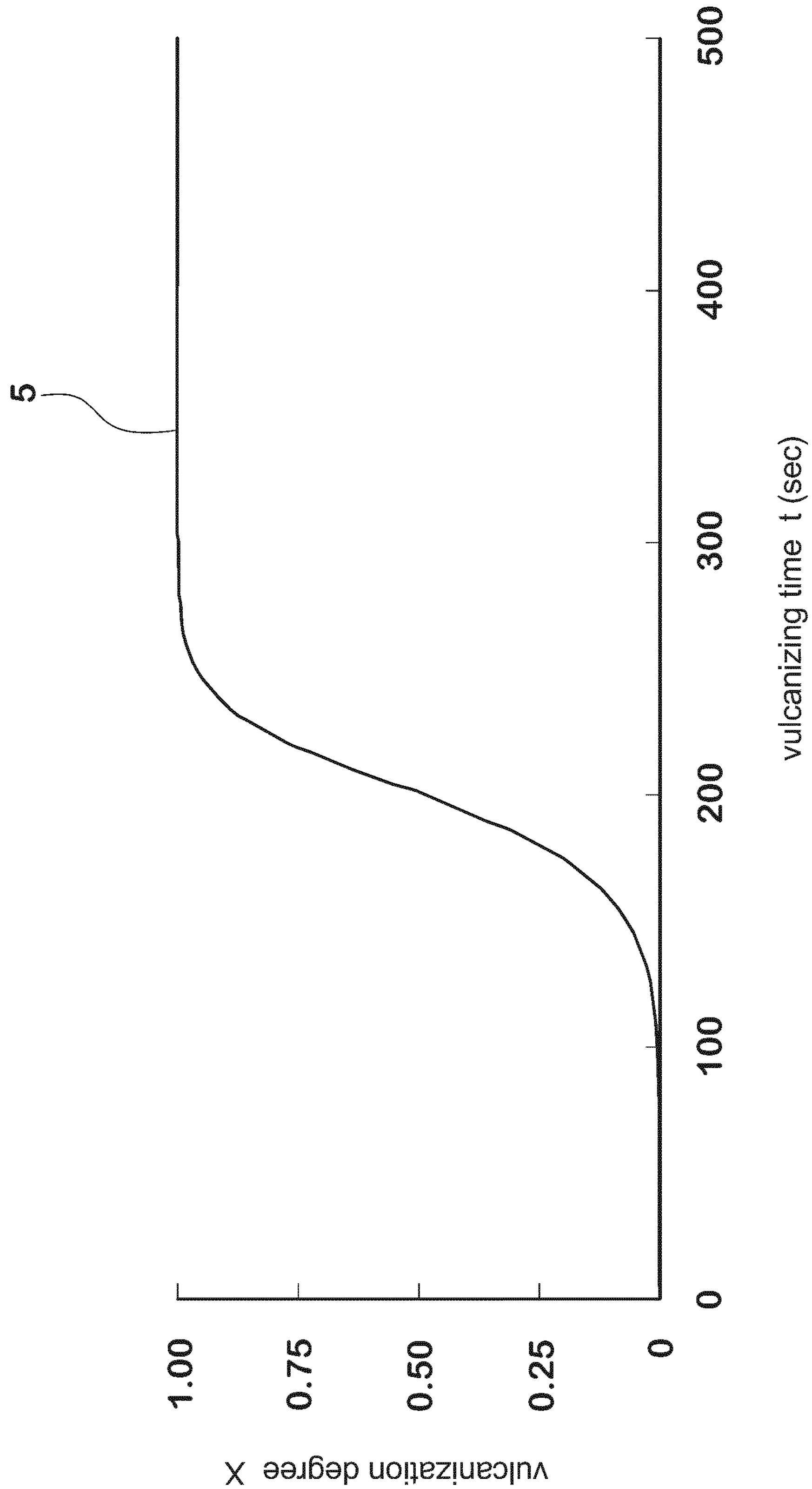
5
vulcanization degree X
1.00
0.75
0.50
0.25
0
0
100
200
300
400
500
vulcanizing time t (sec)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **W.J.TOTH ; J.P.CHANG ; C.ZANICHELLI.** Finite element evaluation of the state of cure in a tire. *Tire Science and Technology,* October 1991, vol. 19 (4), 178-212
- **ARRILAGA et al.** *European Polymer Journal,* 2007, vol. 43, 4783-4799
- **OMRANI et al.** *European Polymer Journal,* 2008, vol. 44, 769-779